# EUROPEAN PATENT SPECIFICATION

(11) **EP 1 490 502 B1**
(45) Date of publication and mention of the grant of the patent: **09.01.2013**
(21) Application number: 02780664.5
(22) Date of filing: 13.11.2002
(51) Int. Cl.: C12Q 1/68

(54) **SCREENING METHOD FOR PREDICTING SUSCEPTIBILITY TO BREAST CANCER**
SCREENING-VERFAHREN ZUR VORHERSAGE DER ANFÄLLIGKEIT FÜR BRUSTKREBS
METHODE DE DEPISTAGE PERMETTANT D'EVALUER UNE PREDISPOSITION AU CANCER DU SEIN

(30) Priority: 14.11.2001 US 332920 P
(43) Date of publication of application: 29.12.2004
(73) Proprietor: Signe BioPharma Inc., Irving TX 75038 (US)
(72) Inventor: Sirbasku, David A., Flower Mound, TX 75028 (US)
(74) Representative: Chapman, Paul Gilmour
(86) International application number: PCT/US2002/036542
(87) International publication number: WO 2003/042660

(56) References cited:
- EP-A- 0 897 981
- WO-A-01/38490
- WO-A-01/62288
- WO-A-01/72846
- WO-A-01/85210
- WO-A-01/86307
- KRAJCI ET AL.: 'The human transmembrane secretory component (poly-Ig receptor): molecular cloning, restriction fragment length polymorphism and chromosomal sublocalization' HUMAN GENETICS vol. 87, 1991, pages 642 - 648, XP000567174
- LOUPART M L ET AL: "ALLELIC IMBALANCE ON CHROMOSOME 1 IN HUMAN BREAST CANCER. I. MINISATELLITE AND RFLP ANALYSIS", GENES, CHROMOSOMES & CANCER, XX, XX, vol. 12, no. 1, 1 January 1995 (1995-01-01), pages 16-23, XP009070345,

## Description

### Field of the Invention

The present invention generally relates to genetic changes associated with the onset of sporadic breast cancer, and more particularly to methods for identifying such changes and for screening women at risk of developing the disease.

### Description of Related Art

The number of breast cancer cases diagnosed each year worldwide is about one million (1). Breast cancer represents 18% of all cancers in women and, with the exception of skin cancer, is the most common site of cancer in women. In decreasing order, the incidence of other cancers in women are cervix, colon/rectum, stomach, endometrium, lung, ovary, mouth/pharynx, esophagus and lymphoma (1). The fact that a similar pattern is widespread throughout the Western world suggests that related genetic changes may be the origin of cancer development at these sites.

Many risk factors have been defined for breast cancer (1-10). When specific risks are given relative ranks, an interesting pattern emerges. The major four risk factors are age (relative risk 10) followed by geographic location, previous breast cancer, and previous benign breast disease (relative risks of 4 to 5) (1). Reproductive history is next most important with relative risks of 2 to 3 (1). Other factors such as diet, alcohol consumption, socioeconomic group and family history are in the relative risk range of 1.3 to 2 (1). Notably, the risk of oral contraceptives or hormone replacement therapy is low, in the relative range of 1.2 to 1.4 (1). From these data, it can be concluded that the origin of breast cancer, and hence the number of causative genes, are yet to be identified. While modification of personal habits, reproductive considerations, and behavior can reduce risk, the benefits are modest and certainly offer no guarantees. Thus far, traditional epidemiology has not provided "the" origin of breast cancer. Indeed, based on epidemiological data, it has even been suggested that breast cancer arises from a single cause (11). But this conclusion came with the statement that the cause was still unknown. With the growing importance of genetic analysis, it now appears likely that epidemiology will move into this arena to make further advances.

Other investigators have made observations that are critical to understanding the genetic origins of breast cancer. First, it is clear that only a small minority of cancers originate from germ-line mutations (12). Second, there are more than 100 changes in gene expression detectable in breast cancer cells versus normal breast epithelium (12). This large number promises to increase to 200 to 300 with new technology (13,14). Based on a recent discussion (12), cancer mutations have been defined as Class I, which involve changes in gene DNA sequences, and Class II genetic alterations, in which changes in gene expression are detected by mRNA analysis (12). By selecting specific RNA species for further study, information can be gained, but there is no assurance which change(s) is causative. Indeed, it seems highly unlikely that cancer development requires this huge number of gene alterations. It is more likely that most of the changes are the result of malignant transformation, not the cause. It may be possible to use mass gene expression analysis (e.g. microarray technology) to predict breast cancer risk or susceptibility, but for now this seems distant.

It seems reasonable to retreat from these types of "shotgun" analysis and approach the issue from another perspective. In this proposal, focus is placed on genetic changes that are more subtle and are represented by loss of heterozygosity (LOH) or other allelic imbalances (AI). These genetic alterations are known to be associated with a high risk of cancer development. There are categories of women with three or more times higher risk of developing breast cancer than average. They are often classified into a group termed "familial breast cancer". They have (i) a first degree relative with breast or ovarian cancer, (ii) one first degree relative with disease diagnosed under the age of 40, (iii) two first or second degree relatives with breast or ovarian cancer under the age of 60, or (iv) three first or second degree relatives with breast or ovarian cancer on the same side of the family (1). It is tempting to conclude that this represents an inherited trait or at least a propensity to development of the disease. This conclusion must be tempered however by an understanding that "inherited" might include as yet unrecognized non-biological inheritance such as culture inheritance and common environmental conditions (15). While genetic predisposition remains a strong possibility, it may not be of the type seem with BRCA1 (16) and BRCA2 (17) which are inherited as autosomal dominants from either parent, albeit with varying penetrance. Equally, it is possible that the predisposition is a recessive inheritance, also with varying penetrance, or a recessive mutation that leads to breast cancer development. It may also represent inherited or acquired genetic abnormalities such as loss of heterozygosity (LOH) or other allelic imbalances (AI) such as abnormal gene numbers.

A familial aspect to breast cancer has been recognized for some time. However, this term may have different meanings. It might indicate "familial clustering" which is the existence of several cases in an extended family. Because breast cancer will occur in 10% of women, chance alone will allow some familial clusters depending upon the number of females in a related group. Therefore, because a family has more than one member with the disease, it does not necessarily follow that there is a genetic cause. In contrast, the familial aspect of breast cancer might also indicate "familial aggregation" which is increased risk to close relatives of women with breast cancer compared to relatives of women without the disease. Familial aggregation is another matter. Its existence may depend on genetic and/or non-genetic causes. As discussed above, the presence of breast cancer in one or more first degree relatives increases risk significantly. As the number of breast cancers in first and second degree relatives increases, especially at younger ages, there is strong reason to hold that there are familial factors that may be genetic. If genetic in origin, identification of these genes will be a major advance in understanding breast cancer (18). Genes which show LOH or AI are strong candidates for identification. The composition and methods of identification of such genes is a primary focus of this disclosure.

The question is: "What genes are being sought to explain familial aggregation"? The low frequency of BRCA1 and BRCA2 does not support the view that they account for one million new cases each year worldwide (18). Although somewhat difficult to assess, one report indicates an estimated frequency of significant mutations in these two genes in the US and UK of 0.0005 to 0.002 (19). Another report (25) states mutations in 1 in 152 and 1 in 833 for mutations in these two genes. It is higher in some regions of the world such as Scandinavia and approaches 1 in 40 in Ashkenazi Jews (20). The major issues resulting from the lack of broad application of BRCA1 and BRCA2 are what other genes are to be sought and by what methods.

There are three separate genetic syndromes that are associated with above average rates of breast cancer development (26). These are Li-Fraumeni Syndrome, Cowden's Syndrome and Ataxia Telangiectasia (AT). Each appears to involve a different gene lesion or small set of lesions, but nonetheless all contribute to a higher risk of breast cancer. These syndromes illustrate the point that a small number of critical mutations are likely involved in breast cancer development. In addition, Lynch Syndrome II is associated with breast cancer development (28). The genetic lesion is important in Lynch Syndrome because it involves impairment of the critical process of DNA mismatch repair (29,30).

Li-Fraumeni Syndrome (LFS) is a very rare germ-line mutation in p53 (31) that increases premenopausal breast cancer as well as sarcomas, brain tumors, leukemia and adrenocortical cancer (32,33). LFS is an autosomal dominant syndrome for which genetic testing is not yet available. It is thought that availability would not change medical management (34). Nonetheless, p53 mutations occur in 30% of sporadic breast cancers (27). The very diverse types of p53 mutations in breast cancer pose a problem for genetic testing (27). One study suggests that mutations at codon 248 might be associated with higher breast cancer risks (35). While screening for P53 changes alone may not be productive, combination screening of P53 with other genes may be very informative based on the accepted view that cancer development is a multistep process involving a relatively few genes.

Cowden's Syndrome is characterized by excess breast cancer, gastrointestinal malignancies, thyroid disease, and other benign conditions (36). Cowden's syndrome carries a lifetime breast cancer risk of 25 to 50%. There is usually early onset and often appearance of bilateral disease (37). There is a germ-line mutation in PTEN, a protein tyrosine phosphatase with homology to tensin. PTEN acts as a tumor suppressor and functions in the control of the cell cycle (38). As with P53, screening for PTEN mutations alone may not be informative, but in combination with other genes, may provide important prognostic value.

Ataxia Telangiectasia (AT) is an autosomal recessive disorder characterized by neurologic deterioration, telangiectasias, immunodeficiencies, and marked hypersensitivity to ionizing radiation. Approximately 1% of the population may be heterozygote carriers of the AT mutation (ATM). Over 200 mutations have been identified in the ATM. The majority are truncations (39). ATM proteins have a role in cell cycle control (40). ATM individuals are susceptible to cancer. Studies have shown that even heterozygotes are at elevated risk for breast cancer (41,42). This is the case despite the fact that the ATM is not identified in breast cancer specimens in excess of its occurrence in control populations (43-45). The link between ATM and breast cancer may be related to the kinase coded for by the AT gene. Its absence leads to chromosomal instability, a condition often associated with breast cancer. Because of the relative high frequency of heterozygotes in the U.S. and European populations, analysis of the AT gene plus other genes will be useful.

Lynch Syndrome II is a form of several related diseases first reported as "cancer family syndrome" (46). Other types of this disease are Lynch Syndrome I, (also called hereditary nonpolyposis colon cancer - HNPCC), Miur's Syndrome (also called Torre's Syndrome), Down family Syndrome, Bloom's Syndrome and finally Dyskeratosis congenital (46). For the purposes of example, Lynch Syndrome II will be discussed. This discussion is intended to encompass these other related forms of the disease as they relate to breast cancer development. Lynch Syndrome II is accompanied by the aggregation of colon, endometrial, ovarian and breast cancer in families (28). This disease is due to mutations in DNA mismatch repair genes designated hMSH2 and hMLH1 (47). The result of these mutations is to create microsatellite instability (48). Microsatellite instability is used today to measure the mutations in populations of specimens from colon cancer patients (49). This has given a range of estimates of the size of population bearing these mutations. It seems likely that the two mutations lead to the accumulation of mutations throughout the genome. With time, genes important in growth regulation of mucosal) cells become altered and result In the onset of cancer.

Cancer is now generally thought to be a multistep disease that arises in response to genetic changes altering key regulatory proteins within the cells. The mutations leading to cancer can be present in the germ line or can arise as somatic mutations in the tissues. It is clear that a progression exists whereby normal cells change to arrive at the fully malignant state capable of metastasizing to distant body sites. While many gene expression changes can be detected by sophisticated technology, it is reasonable to conclude that the powerful methods applied mask the fact that only a relative few changes ultimately result in cancer.

Although advancement has been made toward understanding genetic predisposition to development of certain breast cancers, there remains a pressing need for ways to identify the genetic changes associated with the onset of sporadic breast cancers, which represent about 60-70% of the total number of cases diagnosed each year that have no known genetic origin. There is also a great need for ways to screen individuals for risk of developing the disease and for taking appropriate preventative measures.

### SUMMARY OF PREFERRED EMBODIMENTS

The present invention addresses the problem of sporadic breast cancer, which represents about 60 to 70% of the total cases diagnosed each year and has no known genetic origin. New methods and compositions are provided which employ a newly identified Poly- Ig (Fc) receptor or a Poly-Ig-like (Fc) receptor that is characterized by, among other things, its ability to mediate the recently discovered cell growth inhibitory function of the secretory immune system (e. g., dimeric/polymeric IgA and polymeric IgM). These discoveries are described in detail in U. S Patent Application Nos. 09/852,547 and 09/852,958, and in International Patent Application Nos. PCT/US01/15171 and PCT/US01/15183, the disclosures of which are hereby incorporated herein by reference.

In accordance with the claims, methods for carrying out genetic analysis of the Poly-Ig (Fc) receptor or a Poly-ig-like (Fc) receptor to determine breast cancer susceptibility in heterozygotes and homozygotes are provided. In some embodiments of the present invention, gene screening methods are provided for identifying women at risk of developing the disease either through a familial pattern or a sporadic pattern, In accordance with the invention, methods for double screening an individual for Poly-Ig (Fc) receptor or Poly-Ig-like (Fc) receptor mutations and for mutations of other breast cancer predisposing genes are provided. In certain embodiments of the invention, methods for screening an individual for heterozygous ATM mutations are provided which include comparing the results to screening results for mutations in the Poly-Ig (Fc) receptor or Poly-Ig-like (Fc) receptor.

In some embodiments, the methods comprise screening breast cancer specimens for mutations in growth regulating genes. In another embodiment of the present invention, the methods comprise evaluating breast fluid derived cells for molecular changes in predetermined genes. In some embodiments, premalignant changes in the Poly-Ig (Fc) receptor or a Poly-Ig-like (Fc) receptor gene are assessed, and changes in other predetermined breast cancer predisposing genes are also evaluated.

Also provided in accordance with the present invention is an analytic method that permits a determination of breast cancer susceptibility or risk of future disease. The method includes genetic testing for loss of heterozygosity (LOH) and other Allelic Imbalances (Al) in the poly-Ig-(Fc) receptor or poly-Ig-like (Fc) receptor gene formed in the D1S58 locus of chromosome 1. In some embodiments, the analysis is done with blood cells or mucosal scraping cells to test for germline alterations. For example, analyses are carried out with samples from young women or those at risk because of first or second degree relative breast cancer patterns. Performing early genetic analysis is highly desirable for identifying women at risk. If germline mutations are found or if somatic mutations are found, an appropriate "risk reduction" or "prevention" technology as described in U. S. Patent Application No. 09/852,547 and in International Patent Application No. PCT/US01/15171 (also identified as item 21 in the References, below).

In other embodiments, similar analyses are done with breast fluid derived cells to identify somatic mutations. In this way somatic changes are revealed that may be classified as "pre-malignant" and hence useful in identifying risk In the relative near future. In other embodiments, genetic analysis of breast tumors are carried out as an aid to determining the type of cancer present and indicating if immune therapy is appropriate.

These and other embodiments, features and advantages of the present invention will become apparent with reference to the following description.

### DETAILED DESCRIPTION OF PREFERRED EMBODIMENTS

Recent discoveries disclosed in co-owned, co-pending U. S Patent Application Nos. 09/852,547 and 09/852,958, and in International Patent Application Nos. PCT/US01/15171 and PCT/US01/15183 (also identified as items 21 and 22 in the References, below), each of which is hereby incorporated herein by reference, are expected to lead to resolution of the problem of how to identify genes that are key to the 60 to 70% of breast cancer cases that are today termed"sporadic. "The isolation of new"serum factor (s)" that regulate estrogen responsive breast cancer cell growth in culture is described in the preceding applications. The purification yielded dimeric/polymeric immunoglobulin A (IgA) and pentameric immunoglobulin M (IgM) as the active regulators. These immunoglobulins ("immunoglobulin inhibitors") arrested estrogen target tumor cell growth completely at low nanomolar concentrations, and their inhibitory effects were entirely reversible by picomolar concentrations of estrogens. That disclosure revealed a previously unknown function for the secretory immune system. In the above-identified patent applications, a major role for TGF (3 in breast growth regulation is also identifies: it Is a cytokine that controls IgA/IgM Immunocytes. Breast cancer growth Is best defined as negative paracrine control by secretory immunoglobulins (immunoglobulin inhibitors) and positive direct control by estrogens. In conjunction with this work, the longstanding problem of the regulation of estrogen dependent cell growth in culture under serum-free defined medium conditions was solved. These results have great physiological relevance. IgA and IgM are secreted by B immunocytes located in the lamina propria of estrogen target tissues including breast. They are more than 90% of the immunoglobulins secreted into breast milk. The positioning of the immunocytes in the tissue adjacent to the epithelial cells and the secretion of the immunoglobulins is hormone regulated.

It was found that the secretory immune system products immunoglobulins A and M (IgA and IgM) inhibit the growth of estrogen-sensitive (early) breast cancer by suppressing cell replication. This is the first time a connection was established between the secretory immune system and early estrogen receptor positive (ER⁺) breast cancer growth. In addition, when breast cancer becomes most malignant (*i.e*. ER⁻ hormone-insensitive stages), control by immune system IgA and IgM is lost. Evidence points to the Poly-Ig receptor or a very similar Poly-Ig-like Fc receptor as the mediator of the inhibitory effects of IgA and IgM. This receptor may be the long sought after gene that explains many of the "sporadic" breast cancers. Here, the term "sporadic" refers to breast cancer originating from unknown genetic origins. The Poly-Ig receptor gene is located at locus D1S58 on chromosome 1 (23) which has been proven to be a "hot spot" for allelic imbalances in more than 70% of breast cancers (24). A hot spot is a chromosomal loci or gene that is frequently altered in breast cancer specimens. This site has 46% loss of heterozygosity (LOH) in breast cancer specimens (24) and 30% incidence of allelic imbalance (AI) (24). This research, and that described in the above-identified U.S. and PCT patent applications (21,22), support the conclusion that this gene will have notably broader significance in "sporadic" breast cancer etiology than BRCA1 (located on chromosome 17) or BRCA2 (located on chromosome 13).

Mutations in genes that are critical to cell growth or are known to be predisposing for breast cancer have been described. Such mutations can either cause activation of oncogenes to promote cell replication or cause inactivation of suppressors to release cells to growth without control. In studies related to the present disclosure and described in the above-identified U.S. and PCT patent applications, the Poly-Ig (Fc) receptor or a similar Poly-Ig-like (Fc) receptor has been identified as a tumor suppressor. Binding the ligands IgA or IgM results in growth arrest in ER⁺ breast cancer cells. When this receptor is absent, cells replicate without immune control. The gene identified in this invention has not previously been recognized to have growth regulating properties. As this study developed the application of this new receptor gene function indicated an important concern for both germ line and somatic mutations.

Although the majority of cancers are thought to arise by somatic mutation, the fact that "familial aggregation" of breast cancers exists is strong evidence in favour of germ line analysis. The line of thought is to study both types of mutations in the poly-Ig-like receptor. Germ line mutations will be sought with blood cells or mucosal cells (*i.e*. mucosal scrapings) obtained from women. Somatic mutations are revealed via examination of breast cancer specimens and cells aspirated from breast ducts or in breast fluid samples.

Key models of germ line breast cancer predisposing genes are readily available from sources known to investigators in this field. These include TP53, ATM, PTEN, MLH1 and MSH2 genes. If the full expression of these predisposing genes is to be realized, other mutations must contribute. The Poly-Ig-like receptor is found in every steroid and thyroid hormone responsive cell line examined to date. When the receptor is lost, cells achieve full autonomy. This indicates that the receptor gene may be a key contributor to the development of breast cancer as well as other cancers arising from mucosal tissues.

### Example 1: Genetic Analysis of the Poly-Ig (Fc) receptor or Poly-Ig-like (Fc) Receptor to Determine Breast Cancer Susceptibility in Heterozygotes and Homozygotes

Individuals seeking to determine breast cancer risk for the reasons cited above will be screened for predisposing genetic damage/mutations in the gene for the Poly-Ig (Fc) receptor of Poly-Ig-like (Fc) receptor. This will be done using lymphocytes and technology suited to rapid but accurate screening (e.g. pyrosequencing and rapid PCR methodology). This analysis will be performed similarly to those used to identify heterozygotes and homozygotes for the ATM mutation and for hereditary nonpolyposis colorectal cancer (HNCC). Studies done with control volunteers will be carried out to determine natural innocuous mutations in the gene and to determine if selected populations have different mutations and hence may be at greater risk. It is expected that one damaged gene will confer greater risk than controls because inactivation of the other functional gene will eliminate immune negative growth regulation by IgA and IgM. Suitable techniques that will be used for this genetic analysis are well known in the art. Such an analysis has not been recognized previously as useful in determining risk for breast cancer in populations before development of the disease. Individuals showing homozygous mutations are considered at highest risk and will be counseled to decide on surgical prophylactic measures or on the use of tamoxifen or other anti-estrogen as preventative measures. Thus, genetic screening can be used to not only to define potential risk, but to assistant in initiating preventative life saving actions, as discussed in more detail in the following example.

### Example 2: Screening for Loss of Heterozygosity (LOH) or Allelic Imbalance (AI) In the Poly-Ig (Fc) Receptor or Poly-Ig-like (Fc) Receptor.

Using blood cells, mucosal scrapings, breast fluid derived cells and other body and tissue samples and fluids, the presence of LOH and Al In the Poly-Ig (Fc) receptor or a Poly- Ig-like (Fc) receptor gene will be determined by methods commonly applied and well known. Preferably the D1S58 locus of chromosome 1 will be a primary point of focus. This analysis can begin at very young ages (i. e. nine or ten years old) or can be initiated at age 25 when breast cancer rates are still very low. Analysis of breast fluid cells can be continued with women showing LOH or Al at early ages. This information is used along with methods such as mammography to monitor women at high risk. Early genetic analysis, as described in this example and in Examples 1, 3 and 4, is especially valuable for identifying women at risk so that appropriate steps for risk reduction or prevention can be taken. If germline mutations are found or If somatic mutations are found, the issue then becomes "what to do?". Preventative and therapeutic compositions and methods are described in co-pending U. S. Patent Application No. 09/852,547 and in International Patent Application No. PCT/US01/15171 (also identified as item 21 In the References, below)

### Example 3: Double Screening for Poly-Ig (Fc) Receptor or Poly-Ig-like (Fc) Receptor Mutations and Mutations in other Breast Cancer Predisposing Genes.

Because the development of cancer most likely depends on more than one mutation (50), and may involve several cell types (50), it is useful to screen for mutations in genes that will lead to damage In other genes. Both the Lynch Syndrome II genes and the Cowden's disease gene are candidates for double screening along with the Poly-Ig (Fc) receptor or a Poly-Ig-like (Fc) receptor. For example, changes in the effectiveness of the Lynch Syndrome genes can lead to a gradual accumulation of mutations. If mutations are also present in the Poly-Ig-like receptor, risk can be expected to be substantially higher than controls. Heterozygotes for the Lynch Syndrome genes have not been previously analyzed to determine if they possess an increased risk of breast cancer. Likewise, heterozygotes for the

Cowen's mutation have not been examined before to determine breast cancer susceptibility. These results will then be compared to results of screens for the Poly-Ig (Fc) receptor or Poly-Ig-like (Fc) receptor to identify those individuals at greatest risk. Suitable risk reduction or preventative measures can then be implemented, as discussed in the preceding Example.

### Example 4: Screening for Heterozygous ATM Mutations and Comparison to Mutations in the Poly-Ig (Fc) Receptor or Poly-Ig-like Receptor

The ATM mutation, associated with the disorder ataxia telangiectasia is not found in breast cancers. Nonetheless, it is clear that even heterozyogotes are at substantial risk for breast cancer. In light of the present disclosure of the importance of breast cancer cell growth control by the secretory immunoglobulins, it is of particular significance that the AT disorder is known to be accompanied by chromosome fragility (i.e. lack of repair after ionizing radiation) and a marked deficiency in IgA. Because the ATM mutation is so widely distributed in the population, it is important to initiate a screening for this mutation as a first line defense against breast cancer. Identification of meaningful mutations will then permit decisions by women to elect preventative measures. Pending results indicating a potential problem, these same individuals will then be offered another screening for the Poly-Ig (Fc) receptor as a further indicator of risk status. If significant mutations are found in one or both receptor genes, prophylactic or preventative measures can be initiated, as discussed above in Example 2.

### Example 5: Screening of Breast Cancer Specimens for Mutations in Growth Regulating Genes

Specimens from breast cancer patients will be screened for alterations in the Poly-Ig (Fc) receptor or a Poly-Ig-like receptor to assist in therapy decisions and to identify individuals at greatest risk and requiring more intense intervention. This approach identifies somatic mutations. Conventional screening techniques will also be used to identify heterozygous genes including the Lynch Syndrome gene and the Cowden's disease gene as well as alterations in TP53. The molecular fingerprinting of tumors is expected to increase the effectiveness of treatment programs by allowing each to be adapted to the individual patient. Future use of molecular methods is expected to provide a genetic profile of a patient's primary tumor as well as to provide information relevant to family members concerning their potential risks.

### Example 6: Evaluation of Breast Fluid Derived Cells for molecular Changes in Genes

Cells will be obtained from breast fluids by any of a number of well known methods, or, alternatively by newer methods that are known in the art and have been described in the literature for direct aspiration of the breast milk ducts. Commercial milk pumps are available for this application. Premalignant changes in the Poly-Ig (Fc) receptor or a Poly-Ig-like (Fc) receptor gene will be evaluated as will be changes in the other breast cancer predisposing genes discussed above. This technology is expected to identify somatic mutations at the site of breast cancer development. Identification of sets of changes consistent with long-term development of the disease will permit immediate intervention to eradicate the altered cells or arrest the mutation process.

After parturition, cells will be harvested from expressed breast milk and evaluated for somatic mutations. This analysis can be readily carried out during routine postpartum doctor's office visits. The cells will be collected directly on to filters (supplied) and DNA screening conducted, as previously described. This method permits direct assessment of the genetic status of a subset of reproductive age women without disruption of daily routines. Fluid aspiration can also be done during routine mammography examinations. Nipple pumps, which are available from well known commercial suppliers, can be used to withdraw only the few milliliters of fluid required.

Once a person or a group of persons have been identified as being at risk for breast cancer using one or more of the foregoing procedures, a program of prevention or risk reduction can be Implemented. Such program can include (a) enhancing the amount of an immunoglobulin inhibitor ("immune modulator") of cancer cell growth that contacts the breast ductal tissue of said individuals; and/or administering an immunoglobulin inhibitor mimicking compound; administering an anti-estrogenic compound; (b) administering an aromatase inhibitor. (c) enhancing the number of B immunocytes producing IgA or IgM in breast tissue; and/or (d) immunizing Individuals at risk of developing breast cancer against microorganisms known to or suspected of causing breast cancer. Some immunoglobulin inhibitor mimicking compounds that may be used include: Tamoxifen and MER-25 and chemically substituted or modified derivatives thereof. To reduce possible side effects of MER-25, or its derivative compound, in some cases it may also be desirable to co-administer progesterone or another hormone. Some immune enhancers that may be used include: Levimisole, Imiquimod,
Picibanil, and DHEA. Some useful anti-estrogens include: Tamoxifen, Toremifene, ICI 16384, ICI 182780, EM-800, RU 58688 and EM-139.

### REFERENCES

(1) McPherson K, Steel CM & Dixon JM (2000) Breast cancer - epidemiology, risk factors and genetics. BMJ 321:624-628.
(2) Alberg AJ Helzlsouer KJ (1997) Epidemiology, prevention, and early detection of breast cancer. Current Opinion Oncology 9:505-511.
(3) Kelsey JL & Gammon MD (1990) Epidemiology of breast cancer. Epidemiol Rev 12:228-240.
(4) Adami HO, Signorello LB & Trichopoulos D (1998) Towards an understanding of breast cancer etiology. Semin Cancer Biol 8:255-262.
(5) Petrek JA (1994) Breast cancer and pregnancy. J Natl Cancer Inst Monograph No. 16:113-121.
(6) Kelsey JL & Gammon MD (1991) The epidemiology of breast cancer. CA Cancer J Clin 41:146-165.
(7) Kelsey JL & Bernstein L (1996) Epidemiology and prevention of breast cancer. Annu Rev Public Health 17:47-67.
(8) Lambe M, Hsieh C-C, Trichopoulos D et al (1994) Transient increase in the risk of breast cancer after giving birth. N Eng J Med 331:5-9.
(9) Hulka BS & Moorman PG (2001) Breast cancer: hormones and other risk factors. Maturitas 38:103-106.
(10) Key TJ (1999) Serum oestradiol and breast cancer risk. Endocrine-Related Cancer 6:175-180.
(11) Wiseman RA (2000) Breast cancer: a single cause for the majority of cases. J Epidemiol Community Health 54:851-858.
(12) Sager R (1997) Expression genetics in cancer: shifting the focus from DNA to RNA. Proc Natl Acad Sci USA 94:952-955.
(13) Zhang L, Zhou W, Velculescu VE et al (1997) Gene expression profiles in normal and cancer cells. Science 276:1268-1272.
(14) Monni O, Hyman E, Moussess S et al (2001) From chromosomal alterations to target genes for therapy: integrating cytogenetic and functional genomic views of the breast cancer genome. Semin Cancer Biol 11:395-401.
(15) Morton NE (1982) Outline of Genetic Epiemiology, Karger, Basel.
(16) Miki Y, Swensen J, Shaattuck-Eidens D et al (1994) A strong candidate for the 17-linked breast and ovarian cancer susceptibility gene BRCA1. Science 266:66-71.
(17) Wooster R, Bignell G, Lancaster K et al (1995) Identification of the breast cancer susceptibility gene BRCA2. Nature 378:789-792.
(18) Hopper JL (2001) Genetic epidemiology of female breast cancer. Semin Cancer Biol 11:367-374.
(19) Easton DF (1999) How many more breast cancer predisposition genes are thee? http://breast-cancer-research.com/vol1no1/23 aug99/editorial/1
(20) Strewing JP, Hartge P, Wacholdrer S et al (1997) The risk of cancer associated with specific mutations of BRCA1 and BRCA2 among Ashkenazi Jews. N Eng J Med 336:1401-1408.
(21) Sirbasku, David A. "Compositions and Methods for the Diagnosis, Treatment and Prevention of Steroid Hormone Responsive Cancers" U.S. Patent Application No. 09/852,547 (U.S. Published Application No. 20020006630) and corresponding PCT Published Application No. WO 01/86307.
(22) Sirbasku, David A. "Compositions and Methods for Demonstrating Secretory Immune System Regulation of Steroid Hormone Responsive Cancer Cell Growth" U.S. Patent Application No. 09/852,958 (U.S. Published Application No. 20020012954 and corresponding PCT Published Application No. WO 01/85210.
(23) Krajci P, Gedde-Dahl T, Hoyheim B, et al (1992) The gene encoding human transmembrane secretory component (locus poly-Ig receptor) is linked to D1S58 on chromosome 1. Human Genet 90:215-219.
(24) Loupart M-L, Armour J, Walker R et al (1995) Allelic imbalance on chromosome 1 in human breast cancer. I. Minisatellite and RFLP analysis. Genes, Chromosomes & Cancer 12:16-23.
(25) Iau PT, Macmillan RD & Blamey RW (2001) Germ line mutations associated with breast cancer susceptibility. Eur J Cancer 37:300-321.
(26) Cancer Net, A service of the National Cancer Institute (2001). Genetics of Breast and Ovarian Cancer, pp 1-54. http://cancernet.nci.nih.gov/
(27) Oliver M & Hainaut P (2001) TP53 mutation patterns in breast cancers: searching for clues of environmental carcinogenesis. Semin Cancer Biol 11:353-360.
(28) Nelson CL, Sellers TA, Rich SS et al (1993) Familial clustering of colon, uterine, and ovarian cancers as assessed by family history. Genet Epidemiol 10:235-244.
(29) Lynch HT & Lynch JF (1998) Genetics of colon cancer. Digestion 59:481-492.
(30) Lu KH & Broaddus RR (2001) Gynecological tumors in hereditary nonpolyposis colorectal cancer: we know they are common - now what? Gynecologic Oncology 82:221-222.
(31) Garber JE, Goldstein AM, Kantor AF et al (19910 Follow-up study of twenty-four families with Li-Fraumeni syndrome. Cancer Res 51:6094-6097.
(32) Bottomley RH & Condit PT 919680 Cancer families. Cancer Bulletin 20:22-24.
(33) Malkin D (1993) The Li-Fraaumeni syndrome. Cancer: Principles of Oncology Updates 7:1-14.
(34) Eng C, Hampel H & de la Chapelle A (2001) Genetic testing for cancer predisposition. Annu Rev Med 52:371-400.
(35) Eng C, Schneider K, Fraumeni JF & Li FP (1997) Third international workshop on collaborative interdisciplinary studies of p53 and other predisposing genes in LI-Fraumeni syndrome. Cancer Epidemiol Biomark Prevent 6:379-383.
(36) Tsou HC, Teng DH, Ping XL, et al (1997) The role of MMAC1 mutations in early-onset breast cancer: causative in association with Cowden syndrome and excluded in BRCA1-negative cases. Am J Hum Genet 61:1036-1043.
(37) Olopade OI & Weber BL (1998) Breast cancer genetics: toward molecular characterization of individuals at increased risk for breast cancer: part I. Cancer: Principles and Practice of Oncology Updates 12:1-12.
(38) Lynch ED, Ostermeyer EA, Lee MK et al (1997) Inherited mutations in PTEN that are associated with breast cancer, Cowden disease, and juvenile polyposis. Am J Hum Genet 61:1254-1260.
(39) Telatar M, Teroka S, Wang Z et al (1998) Ataxia-telangiectasia gene with a product similar to PI-3 kinase. Science 268:86-97.
(40) Uhrhammer N, Bay JO, Bignon YJ et al (1998) Seventh international workshop on ataxia-telangiectasia. Cancer Res 58:3480-3485.
(41) Swift M, Reitnauer PJ, Morrell D et al (1987) Breast and other cancers in families with ataxia-telangiectasia. N Eng J Med 316:1289-1294.
(42) Easton DF (1994) Cancer risks in A-T heterozygotes. Int J Radiation Biol 66:S177-S182.
(43) Fitzgerald MG, Bean JM, Hegde SR et al (1997) Heterozygous ATM mutations do not contribute to early onset of breast cancer. Nature Genetics 15: 307-310.
(44) Chen J, BirKholtz GG, Lindblom P et al (1998) The role of ataxia0telangiectasia heterozygotes in familial breast cancer. Cancer Res 58: 1376-1379.
(45) Bay JO, Grancho M, Pemin D et al (1998) No evidence for constitutional ATM mutation on breast/gastric cancer families. Int J Oncol 12: 1385-1390.
(46) Gastrointestinal Pathology, An Atlas and Text, Fenoglio-Preiser CM (ed), Lippincott-Raven, 2nd Edition, (1999); Chapter 20: Carcinomas and other Epithelial and Neuroendocrine Tumors of the Large Intestine, pp 9091068.
(47) Lynch HT, Watson P, Shaw TG et al (1999) Clinical impact of molecular genetic diagnosis, genetic counseling, and management of hereditary cancer. Part II. Hereditary nonpolyposis colorectal carcinoma as a model. Cancer 86 (suppl 11): 2457-2463.
(48) Atkin NB (2001) Microsatellite instability. Cytogenet Cell Genet 92: 177-181.
(49) Percesepe A, Borghl F, Menigatti M et al (2001) Molecular screening for hereditary nonpolyposis colorectal cancer a prospective, population-based study. J Clin Oncol 19: 3944-3950.
(50) Beckmann MW, Niederacher D, Schnurch H-G et al (1997) Multistep carcinogenesis of breast cancer and tumor heterogeneity. J Mol Med (review) 75: 429-439.

## Claims

1. An *in vitro* method to aid in identifying a familial or sporadic pattern of risk in at least one individual for developing breast cancer, the method comprising:
I) screening said at least one Individuals for the presence of a mutation in the Poly-Ig (Fc) receptor,
and further comprising
II) screening for a mutation In at least one additional gene that is associated with breast cancer or with an Increased risk of developing breast cancer.

2. The method of claim 1 wherein said screening comprises determining heterozygosity or homozygosity for the mutation in the Poly-Ig (Fc) receptor.

3. The method according to either of claims 1 or 2 wherein the individual Is being tested for a sporadic pattern of risk.

4. The method according to any preceding claim wherein a germ line and/or somatic mutation in the Poly-Ig (Fc) receptor gene is screened for.

## Patentansprüche

1. In-vitro-Verfahren zur Unterstützung beim Erkennen eines familiären oder sporadischen Risikomusters bei mindestens einer Person für die Entwicklung von Brustkrebs, wobei das Verfahren aufweist:
I) Untersuchen der mindestens einen Person auf die Anwesenheit einer Mutation im Poly-Ig (Fc)-Rezeptor;
und wobei das Verfahren ferner aufweist:
II) Untersuchen auf eine Mutation in mindestens einem weiteren Gen, die mit Brustkrebs oder mit einem erhöhten Risiko der Entwicklung von Brustkrebs assoziiert ist.

2. Verfahren nach Anspruch 1, wobei die Untersuchung die Bestimmung der Heterozygotie oder Homozygotie für die Mutation im Poly-Ig (Fc)-Rezeptor beinhaltet.

3. Verfahren nach Anspruch 1 oder Anspruch 2, wobei die Person auf ein sporadisches Risikomuster getestet wird.

4. Verfahren nach einem der vorstehenden Ansprüche, wobei auf eine Keimbahn- und/oder somatische Mutation im Poly-Ig (Fc)-Rezeptor untersucht wird.

## Revendications

1. Méthode *in vitro* destinée à faciliter l'identification d'un modèle familial ou sporadique du risque, chez un individu au moins, de développer un cancer du sein, la méthode comprenant:
i) dépister chez au moins un individu la présence d'une mutation dans le récepteur Poly-Ig (Fc);
et comprenant en outre
ii) dépister une mutation dans au moins un gène supplémentaire qui est associée au cancer du sein ou à un risque accru de développer un cancer du sein.

2. Méthode selon la revendication 1, dans laquelle ledit dépistage comprend déterminer la nature hétérozygote ou homozygote de la mutation dans le récepteur Poly-Ig (Fc).

3. Méthode selon l'une quelconque des revendications 1 ou 2, dans laquelle l'individu est testé pour un modèle sporadique de risque.

4. Méthode selon l'une quelconque des revendications précédentes, dans laquelle une lignée germinale et/ou une mutation somatique dans le gène du récepteur Poly-Ig (Fc) est dépistée.
